(19) Europäisches Patentamt
European Patent Office
Office européen des brevets

(11) Veröffentlichungsnummer: **0 221 444**
**B1**

(12) # EUROPÄISCHE PATENTSCHRIFT

(45) Veröffentlichungstag der Patentschrift: **19.07.89**

(51) Int. Cl.⁴: **C07D 321/06**, A61K 7/46

(21) Anmeldenummer: **86114625.6**

(22) Anmeldetag: **22.10.86**

(54) **Substituierte 4-Methyl-4,7-dihydro-1,3-dioxepine, deren Herstellung und Verwendung als Riechstoffe.**

(30) Priorität: **07.11.85 DE 3539467**

(43) Veröffentlichungstag der Anmeldung:
**13.05.87 Patentblatt 87/20**

(45) Bekanntmachung des Hinweises auf die Patenterteilung:
**19.07.89 Patentblatt 89/29**

(84) Benannte Vertragsstaaten:
**CH DE ES FR GB IT LI NL**

(56) Entgegenhaltungen:
**EP-A- 0 024 473**
**EP-A- 0 085 937**
**DE-B- 2 407 863**
**US-A- 557 949**
**US-A- 3 769 303**

(73) Patentinhaber: **BASF Aktiengesellschaft, Carl-Bosch-Strasse 38, D-6700 Ludwigshafen(DE)**

(72) Erfinder: **Gramlich, Walter, Dr., Auf der Höhe 11, D-6803 Edingen-Neckarhausen(DE)**
Erfinder: **Siegel, Hardo, Dr., Hans-Purrmann-Allee 25, D-6720 Speyer(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft substituierte 4-Methyl-4,7-dihydro-1,3-dioxepine der allgemeinen Formel I

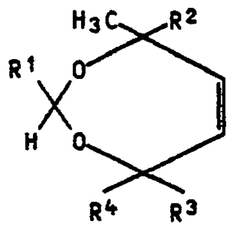

(I),

in der $R^1$ für einen geradkettigen oder verzweigten Alkylrest mit 1 bis 12 C-Atomen, der auch Sauerstoff in Form einer Ethergruppierung enthalten kann oder einen gegebenenfalls durch Alkylgruppen und/oder eine Alkylengruppe substituierten 5 bis 8-gliedrigen Ring mit bis zu 10 C-Atomen, der auch Sauerstoff in Form einer Ethergruppierung enthalten kann, vorzugsweise für einen geradkettigen oder verzweigten Alkylrest mit 1 bis 6 C-Atomen, steht
und $R^2$, $R^3$ und $R^4$ für Wasserstoff oder Methyl stehen, wobei die Gesamt- Kohlenstoffatomzahl maximal 25 beträgt, ausgenommen die Verbindung 2-tert.-Butyl-4,7-dihydro-4,7-dimethyl-1,3-dioxepin.

Wegen der im allgemeinen unzureichenden Verfügbarkeit vieler natürlicher Riechstoffkomponenten, der notwendigen Anpassung an wechselnde modische Geschmacksrichtungen sowie dem ständig steigenden Bedarf an Geruchsverbesserern für Produkte des täglichen Bedarfs, wie Reinigungsmittel, Kosmetika, Leime etc. hat die Riechstoffindustrie ständig Bedarf an neuen Riechstoffen, die allein oder in Form von Kompositionen wertvolle Parfüms bzw. Duftstoffe mit interessanten Duftnoten darstellen. Da aufgrund wenig bekannter Zusammenhänge zwischen Struktur und Riechstoffeigenschaften eine gezielte Synthese von Riechstoffen mit gewünschten olfaktorischen Eigenschaften nicht möglich ist, besteht die Aufgabe, solche Verbindungen aufzufinden, die wertvolle Riechstoffqualitäten besitzen.

Es wurde nun zufällig gefunden, daß die oben beschriebenen Verbindungen der Formel I, d.h. in 2-Stellung substituierte 4,7-Dihydro-1,3-dioxepine, die in 4- oder 7-Stellung mindestens eine Methylgruppe aufweisen, eine neue Klasse mit hochinteressanten Geruchsnoten darstellen.

In 2-Stellung mono- oder di-alkylierte 4,7-Dihydro-1,3-dioxepine (1,3-Dioxa-5-cyclohexene) der Formel IV

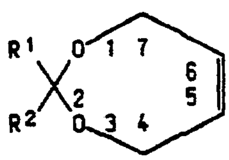

(IV)

($R^1$ und $R^2$ = H oder Alkyl)

sind aus der Literatur (siehe M.J. Soulier u.a., Bull. Soc. Chim. Fr. 1975, Seiten 1763-66) als Herbizide und Polymerisations-Katalysatoren sowie als Vorprodukte für Riechstoffe (siehe EP 24 473) bekannt. Sie selbst besitzen keine interessanten olfaktorischen Eigenschaften und wurden in loc. cit. auch nicht selbst als Riechstoffe erwähnt.

Weiterhin sind das 4,7-Dimethyl-4,7-dihydro-1,3-dioxepin und das 2-tert.-Butyl-4,7-dimethyl-4,7-dihydro-1,3-dioxepin aus einer Arbeit über Konformationsanalysen in J. Org. Chem. Vol. 40, No. 4 (1975), Seiten 450-453 bekannt. Anmerkungen über eventuelle Riechstoffqualitäten finden sich in der Arbeit nicht.

Darüber hinaus sind in 2-Stellung unsubstituiertes 4,4,7,7-Tetramethyl-4,7-dihydro-1,3-dioxepin und 4,7-Dimethyl-4,7-diethyl-4,7-dihydro-1,3-dioxepin aus CA69:P19996a als Komponente für die Herstellung von Co-Polymeren bekannt. Auch in den zuletzt genannten Literaturstellen findet sich keinerlei Hinweis darüber, daß Verbindungen mit der 1,3-Dioxepin-Struktur als Riechstoffe interessant sein könnten.

Es war daher sehr überraschend, daß die erfindungsgemäßen 4-Methyl-4,7-dihydro-1,3-dioxepine Verbindungen mit hochinteressanten Geruchsnoten darstellen und daher als Riechstoffe verwendet werden können.

Weiter ist aus US-A 557 949 das 4,4,7,7–Tetramethyl·2–ethyl–1,3–dioxep–5·in bekannt, das als Zwischenprodukt für die Herstellung von Polyacetalen Verwendung findet, Riechstoffqualitäten sind nich bekannt.

Gegenstand der Anmeldung ist daher auch die Verwendung der substituierten 4-Methyl-4,7-dihydro-1,3-dioxepine der allgemeinen Formel I

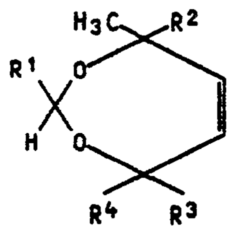

(I).

in der $R^1$ für einen geradkettigen oder verzweigten Alkylrest mit 1 bis 12 C-Atomen, der auch Sauerstoff in Form einer Ethergruppierung enthalten kann oder einen gegebenenfalls durch Alkylgruppen und/oder eine Alkylengruppe substituierten 5 bis 8-gliedrigen Ring mit bis zu 10 C-Atomen, der auch Sauerstoff in Form einer Ethergruppierung enthalten kann, vorzugsweise einen geradkettigen oder verzweigten Alkylrest mit 1 bis 6 C-Atomen, steht,
und $R^2$, $R^3$ und $R^4$ für Wasserstoff oder Methyl stehen, wobei die Gesamt-Kohlenstoffatomzahl maximal 25 beträgt, als Riech- und Aromastoffe sowie Riechstoffkompositionen gekennzeichnet durch einen Gehalt an diesen substituierten 4-Methyl-4,7-dihydro-1,3-dioxepinen.

Gegenstand der Anmeldung ist ferner ein Verfahren zur Herstellung von 4-Methyl-4,7-dihydro-1,3-dioxepinen der allgemeinen Formel I, in der $R^1$ bis $R^4$ die oben angegebene Bedeutung besitzen, das dadurch gekennzeichnet ist, daß man einen Aldehyd der allgemeinen Formel II

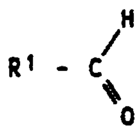

(II),

in der $R^1$ die oben angegebene Bedeutung hat, oder dessen Acetale mit Alkendiolen der allgemeinen Formel III

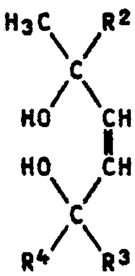

(III),

in der $R^2$ bis $R^4$ die oben angegebene Bedeutung haben, gegebenenfalls in Gegenwart eines sauren Katalysators cyclisiert.

Als geeignete Aldehyde der allgemeinen Formel II seien beispielsweise genannt:

Acetaldehyd, Propionaldehyd, 2-Methyl-propanal, n-Butyraldehyd, 2-Methyl- butanal, 3-Methyl-butanal, 2-Ethyl-butanal, Valeraldehyd, 2-Methyl-pentanal, 3-Methyl-pentanal, 4-Methyl-pentanal, 2,2-Dimethyl-propanal (Pivalinaldehyd) sowie unsubstituierte höhere Aldehyde wie n-Hexanal, n-Heptanal, n-Octanal, n-Nonanal, Decanal sowie deren ein- und/oder mehrfach alkylierte Derivate; bekannteste Vertreter sind das chemische Großprodukt 2-Ethyl-hexanal sowie 3,5,5-Trimethyl-hexanal.

Anstelle des Aldehyds können auch die entsprechenden Acetale, wie das Dimethylacetal eingesetzt werden. In den meisten Fällen kann man jedoch direkt von der Carbonylverbindung ausgehen.

Als Alkendiole der allgemeinen Formel III können beispielsweise eingesetzt werden:

2-Penten-1,4-diol, 4-Methyl-2-penten-1,4-diol, 3-Hexen-2,5-diol, 2-Methyl-3-hexen-2,5-diol und 2,5-Dimethyl-3-hexen-2,5-diol. Alle Alkendiole sind aus den entsprechenden Alkindiolen leicht durch partielle Hydrierung zugänglich. Die Alkindiole stellen entweder selbst bedeutende chemische Zwischenprodukte dar oder lassen sich leicht durch Umsetzung von Acetylen mit den entsprechenden Carbonylverbindungen erhalten.

Zur Cyclisierung werden meist katalytische Mengen eines sauren Katalysators, wie p-Toluolsulfonsäure, Phosphorsäure, Oxalsäure oder eines sauren Ionenaustauschers eingesetzt.

Die neuen erfindungsgemäßen Verbindungen liegen bei unsymmetrischer Substitution an den C-Atomen 4 und 7 als Diastereomerengemische vor. Aufgrund der abgerundeten Geruchsnoten ist eine Auftrennung vom olfaktorischen Gesichtspunkt aus nicht notwendig. Sie können daher in stereoisomeren Formen sowohl als Diastereomere als auch als Enantiomere vorliegen.

Die neuen 4,7-Dihydro-1,3-dioxepine der Formel I lassen sich gut mit anderen Riechstoffen in verschiedenen Mengenverhältnissen zu neuartigen, interessanten Riechstoffkompositionen kombinieren. Außer in der Feinparfümerie können derartige Kompositionen zur Parfümierung von Kosmetika wie Cremes, Lotionen, Aerosolen, Toilettenseifen, technischen Artikeln wie Reinigungs- und Waschmitteln, Weichspülern, Desinfektionsmitteln und Textilbehandlungsmitteln dienen.

Darüber hinaus haben die 4,7-Dihydro-1,3-diepoxine Bedeutung als Vorprodukte für weitere wertvolle Riechstoffe. Beispielsweise können aus ihnen durch Hydroformylierung die entsprechenden 5-Formyl-4,7-dihydro-1,3-dioxepine hergestellt werden, die eine neue Klasse von hochinteressanten synthetischen Riechstoffen darstellen.

Besonders wertvolle Verbindungen der allgemeinen Formel I sind als Riechstoffe für die Feinparfümerie:

2-Propyl-4,4,7,7-tetramethyl-4,7-dihydro-1,3-dioxepin,
2-tert.-Butyl-4,4,7,7-tetramethyl-4,7-dihydro-1,3-dioxepin,
2,4,4,7,7-Pentamethyl-4,7-dihydro-1,3-dioxepin,
2-Pentyl-4,4,7,7-tetramethyl-4,7-dihydro-1,3-dioxepin,
2-Ethyl-4,4,7,7-tetramethyl-4,7-dihydro-1,3-dioxepin und
2-Propyl-4,7-dimethyl-4,7-dihydro-1,3-dioxepin.

Die folgenden Beispiele sollen den Gegenstand der Erfindung näher erläutern.

<u>Beispiele 1 bis 15</u>

Jeweils ein Gemisch aus 5 Mol des aus Tabelle 1 ersichtlichen Alkendiols, 3 g p-Toluolsulfonsäure, 5 Mol der aus Tabelle 1 ersichtlichen Carbonylverbindung und 1 Liter (L) Toluol oder Cyclohexan wurde für die aus der Tabelle ersichtliche Reaktionszeit unter Rückfluß zum Sieden erhitzt, wobei das anfallende Reaktionswasser aus dem Reaktionsgemisch abgetrennt wurde. Nach dem Abkühlen auf Raumtemperatur (RT) wurde jeweils mit 10 ml einer 25 %igen wäßrigen NaOH versetzt, das Reaktionsgemisch mit wenig Wasser neutral gewaschen, das Toluol bei 50 bis 60°C abdestilliert und der Rückstand unter vermindertem Druck destilliert. Die physikalischen Daten und Geruchsbeschreibungen der erhaltenen substituierten 4,7-Dihydro-4-methyl-1,3-dioxepine sind in Tabelle 1 angegeben.

Tabelle 1

| Beispiel | Alkendiol | Carbonylverbindung | -4,7-dihydro-1,3-dioxepine | KP [°C/mbar] | $n_D^{25}$ | Geruchsbeschreibung |
|---|---|---|---|---|---|---|
| 1 | 3-Hexen-2,5-diol | n-Butanal | 2-Propyl-4,7-dimethyl- | 36/0,4 | 1,4382 | grün, Gras-artig |
| 2 | 2,5-Dimethyl-3-hexen-2,5-diol | n-Hexanal | 2-Pentyl-4,4,7,7-tetramethyl- | 83/0,1 | 1,4450 | grün-krautige Note |
| 3 | 2,5-Dimethyl-3-hexen-2,5-diol | n-Butanal | 2-Propyl-4,4,7,7-tetramethyl- | 66/0,1 | 1,4408 | Campherartig mit Fenchelnote |
| 4 | 2,5-Dimethyl-3-hexen-2,5-diol | 2,2-Dimethyl-propanal | 2-tert.-Butyl-4,4,7,7-tetramethyl- | 47/0,5 | 1,4380 | Minze-Note |
| 5 | 2,5-Dimethyl-3-hexen-2,5-diol | Acetaldehyd | 2,4,4,7,7-Pentamethyl- | 50/0,1 | 1,4362 | L-Menthol-artig, kühlender Effekt |
| 6 | 2,5-Dimethyl-3-hexen-2,5-diol | 3,5,5-Trimethyl-hexanal | 2-(2,4,4-Trimethyl-pentyl)-4,4,7,7-tetramethyl- | 81/0,1 | 1,4442 | frisch, Ozon-artig |
| 7 | 2,5-Dimethyl-3-hexen-2,5-diol | 2-Methyl-propanal | 2-Isopropyl-4,4,7,7-tetramethyl- | 36/0,3 | 1,4381 | Anis-, Fenchel-artig |
| 8 | 3-Hexen-2,5-diol | n-Hexanal | 2-Pentyl-4,7-dimethyl- | 57/0,5 | 1,4437 | krautig, Sellerie-artig |
| 9 | 3-Hexen-2,5-diol | 2-Methyl-undecanal | 2-(1-Methyl-decyl)-4,7-dimethyl- | 130/0,01 | 1,4470 | schwache Grün-Note |
| 10 | 2,5-Dimethyl-3-hexen-2,5-diol | n-Propanal | 2-Ethyl-4,4,7,7-tetramethyl | 34/0,4 | 1,4379 | Basilikum, Estragon-artig |
| 11 | 2,5-Dimethyl-3-hexen-2,5-diol | Methoxy-acetaldehyd | 2-Methoxymethyl-4,4,7,7-tetramethyl- | 46/0,015 | 1,4431 | krautig, fruchtig |
| 12 | 3-Hexen-2,5-diol | 3-Formyl-pinan | 2-(2,7,7-Trimethyl-bicyclo[3,1,1$^{1,5}$]hept-3-yl)-4,7-dimethyl- | 78/0,01 | 1,4841 | Sellerie-artig |
| 13 | 3-Hexen-2,5-diol | 3-Formyl-tetrahydro-pyran | 2-(3-Tetrahydropyranyl)-4,7-dimethyl- | 95/0,01 | 1,4712 | frisch blumig, fruchtig |
| 14 | 3-Hexen-2,5-diol | Cyclopentyl-carbaldehyd | 2-Cyclopentyl-4,7-dimethyl- | 67/0,08 | 1,4661 | krautig, Borneol-artig |
| 15 | 4-Methyl-2-penten-1,4-diol | 2-Methyl-propanal | 2-Isopropyl-4,4-dimethyl- | 45/0,25 | 1,4472 | frische Grüne |

Anwendungsbeispiel

Die großen Einsatzmöglichkeiten der neuen Verbindungen sollen beispielsweise anhand der Wirkung von 2-Pentyl-4,4,7,7-tetramethyl-1,3-dioxacyclohept-5-en (Beispiel 2) in einer Grün-Komposition gezeigt werden (Lit.: F.V. Wells & M. Billot, Perfumery Technology, 2nd Ed., Halsted Press/John Wiley & Sons, New York 1981; Seite 271).

| Green Bouquet 279 | A | B |
| --- | --- | --- |
| 2-Pentyl-4,4,7,7-tetramethyl-4,7-dihydro-1,3-dioxepin | 0 | 7 |
| Phenylacetaldehyd 100% | 1,5 | 1,5 |
| Phenylacetaldehyd-dimethylacetal | 7 | 0 |
| p-Toluyl-acetaldehyd | 5 | 5 |
| Cetone V (Allylionon) | 3 | 3 |
| cis-3-Hexenol | 3 | 3 |
| Methylphenylcarbinylacetat | 5 | 5 |
| Eichenmoos abs. | 2 | 2 |
| Zimtsäuremethylester | 3 | 3 |
| Zimtalkohol | 5 | 5 |
| Methylnaphthylketon | 2 | 2 |
| Hydroxycitronellal | 6 | 6 |
| Dimethylbenzylcarbinylacetat | 5 | 5 |
| Lilial (Givaudan) | 3 | 3 |
| Phenylethylalkohol | 10 | 10 |
| Citronellol | 6 | 6 |
| Phenylethylacetat | 3 | 3 |
| Iso-Eugenol | 3 | 3 |
| Ylang-ylang-Öl | 5 | 5 |
| Undecylenaldehyd 10% | 1 | 1 |
| Cyclamenaldehyd | 3 | 3 |
| Bergamott-Öl | 3 | 3 |
| Lemon-Öl, Guinea | 1,5 | 1,5 |
| Galbanum-Öl | 1 | 1 |
| Elemi-Öl | 1 | 1 |
| Methyl-heptin-carbonat 5% | 2 | 2 |
| Jasmin-Base SR | 10 | 10 |
| | 100 | 100 |

Der Ersatz des Phenylacetaldehyddimethylacetals im Green Bouquet 279 (A) durch die neue Verbindung aus Beispiel 4 gibt dem grünen Fantasie-Akkord eine noch frischere, rundere würzige Note.

**Patentansprüche**

1. Substituierte 4-Methyl-4,7-dihydro-1,3-dioxepine der allgemeinen Formel I

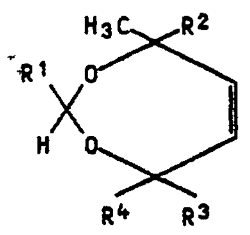

(I),

in der $R^1$ für einen geradkettigen oder verzweigten Alkylrest mit 1 bis 12 C-Atomen, der auch Sauerstoff in Form einer Ethergruppierung enthalten kann oder einen gegebenenfalls durch Alkylgruppen und/oder eine Alkylengruppe substituierten 5 bis 8-gliedrigen Ring mit bis zu 10 C-Atomen, der auch Sauerstoff in Form einer Ethergruppierung enthalten kann, steht,

und $R^2$, $R^3$ und $R^4$ für Wasserstoff oder Methyl stehen, wobei die Gesamt-Kohlenstoffatomzahl maximal 25 beträgt, ausgenommen die Verbindungen 2-tert.-Butyl-4,7-dihydro-4,7-dimethyl-1,3-dioxepin und 4,4,7,7-Tetramethyl-2-ethyl-1,3-dioxep-5-in.

2. Verwendung der substituierten 4-Methyl-4,7-dihydro-1,3-dioxepine der allgemeinen Formel I

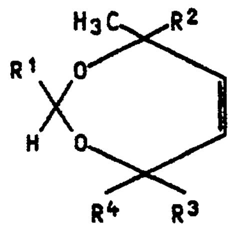

(I),

in der $R^1$ für einen geradkettigen oder verzweigten Alkylrest mit 1 bis 12 C-Atomen, der auch Sauerstoff in Form einer Ethergruppierung enthalten kann oder einen gegebenenfalls durch Alkylgruppen und/oder eine Alkylengruppe substituierten 5 bis 8-gliedrigen Ring mit bis zu 10 C-Atomen, der auch Sauerstoff in Form einer Ethergruppierung enthalten kann, steht,
und $R^2$, $R^3$ und $R^4$ für Wasserstoff oder Methyl stehen, wobei die Gesamt-Kohlenstoffatomzahl maximal 25 beträgt,
als Riech- und Aromastoffe.

3. Riechstoffkompositionen, gekennzeichnet durch einen Gehalt an den substituierten 4-Methyl-4,7-dihydro-1,3-dioxepinen der allgemeinen Formel I gemäß Anspruch 2.

4. Riechstoffkompositionen nach Anspruch 3, dadurch gekennzeichnet, daß sie die 4-Methyl-4,7-dihydro-1,3-dioxepine in einer Menge von 0,5 bis 50 Gew.-% enthalten.

5. 2-Propyl-4,4,7,7-tetramethyl-4,7-dihydro-1,3-dioxepin.

6. 2-tert.-Butyl-4,4,7,7-tetramethyl-4,7-dihydro-1,3-dioxepin.

7. 2,4,4,7,7-Pentamethyl-4,7-dihydro-1,3-dioxepin.

8. 2-Pentyl-4,4,7,7,-tetramethyl-4,7-dihydro-1,3-dioxepin.

9. 2-Ethyl-4,4,7,7-tetramethyl-4,7-dihydro-1,3-dioxepin.

10. 2-Propyl-4,7-dimethyl-4,7-dihydro-1,3-dioxepin.

11. 2-Pentyl-4,7-dimethyl-4,7-dihydro-1,3-dioxepin.

## Claims

1. A substituted 4-methyl-4,7-dihydro-1,3-dioxepin of the formula I

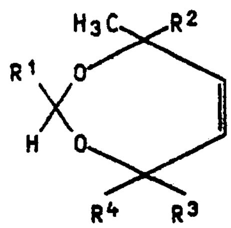

(I),

where $R^1$ is a straight-chain or branched alkyl radical of 1 to 12 carbon atoms which may furthermore contain oxygen in the form of an ether group, or is a 5-membered to 8-membered ring which is unsubstituted or substituted by alkyl groups and/or an alkylene group, has up to 10 carbon atoms and may furthermore contain oxygen in the form of an ether group, and $R^2$, $R^3$ and $R^4$ are each hydrogen or methyl, the total number of carbon atoms being not more than 25, with the exception of the compounds 2-tert-butyl-4,7-dihydro-4,7-dimethyl-1,3-dioxepin and 4,4,7,7-tetramethyl-2-ethyl-1,3-dioxep-5-in.

2. Use of the substituted 4-methyl-4,7-dihydro-1,3-dioxepin of the formula I

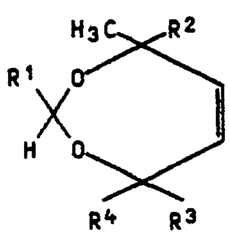

(I),

where $R^1$ is a straight-chain or branched alkyl radical of 1 to 12 carbon atoms which may furthermore contain oxygen in the form of an ether group, or is a 5-membered to 8-membered ring which is unsubstituted or substituted by alkyl groups and/or an alkylene group, has up to 10 carbon atoms and may furthermore contain oxygen in the form of an ether group, and $R^2$, $R^3$ and $R^4$

3. A scent composition which contains a substituted 4-methyl-4,7-dihydro-1,3-dioxepin of the formula I as claimed in claim 2.

4. A scent composition as claimed in claim 3, which contains a 4-methyl-4,7-dihydro-1,3-dioxepin in an amount of from 0.5 to 50% by weight.

5. 2-Propyl-4,4,7,7-tetramethyl-4,7-dihydro-1,3-dioxepin.

6. 2-tert-Butyl-4,4,7,7-tetramethyl-4,7-dihydro-1,3-dioxepin.

7. 2-4,4,7,7-Pentamethyl-4,7-dihydro-1,3-dioxepin.

8. 2-Pentyl-4,4,7,7-tetramethyl-4,7-dihydro-1,3-dioxepin.

9. 2-Ethyl-4,4,7,7-tetrametyl-4,7-dihydro-1,3-dioxepin.

10. 2-Propyl-4,7-dimethyl-4,7-dihydro-1,3-dioxepin.

11. 2-Pentyl-4,7-dimethyl-4,7-dihydro-1,3-dioxepin.

**Revendications**

1. 4-méthyl-4,7-dihydro-1,3-dioxépines substituées de formule générale I

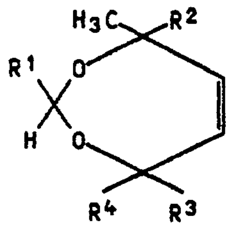

(I).

dans laquelle $R^1$ représente un reste alkyle à chaîne droite ou ramifiée, de 1 à 12 atomes C, qui peut contenir aussi de l'oxygène sous forme d'un groupement éther ou un noyau de 5 à 8 chaînons, ayant jusqu'à 10 atomes C, éventuellement substitué par des groupes alkyle et/ou un groupe alkylène, qui peut aussi contenir de l'oxygène sous forme d'un groupement éther et $R^2$, $R^3$ et $R^4$ sont mis pour hydrogène ou méthyle, le nombre total d'atomes de carbone s'élevant au maximum à 25, à l'exception des composés 2-tert.-butyl-4-dihydro-4,7-diméthyl-1,3-dioxépine et 4,4,7,7-tétra-méthyl-2-éthyl-1,3-dioxep-5-ine.

2. Utilisation, comme matières premières odorantes et parfums, des 4-méthyl-4,7-dihydro-1,3-dioxépines substituées de formule générale I

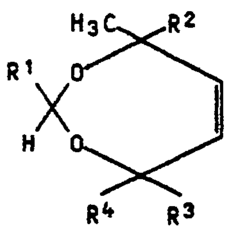

(I).

dans laquelle $R^1$ représente un reste alkyle à chaîne droite ou ramifiée, de 1 à 12 atomes C, qui peut contenir aussi de l'oxygène sous forme d'un groupement éther ou un noyau de 5 à 8 chaînons, ayant jusqu'à 10 atomes C, éventuellement substitué par des groupes alkyle et/ou un groupe alkylène, qui peut aussi contenir de l'oxygène sous forme d'un groupement éther et $R^2$, $R^3$ et $R^4$ sont mis pour hydrogène ou méthyle, le nombre total d'atomes de carbone s'élevant au maximum à 25.

3. Compositions de matières odorantes, caractérisées le fait qu'elles contiennent des 4-méthyl-4,7-dihydro-1,3-dioxépines de formule générale I selon la revendication 2.

4. Compositions de matières odorantes selon la revendication 3, caractérisées par le fait qu'elles contiennent les 4-méthyl-4,7-dihydro-1,3-dioxépines en quantités de 0,5 à 50% en poids.

5. 2-propyl-4,4,7,7-tétraméthyl-4,7-dihydro-1,3-dioxépine.

6. 2-tert.-butyl-4,4,7,7-tétraméthyl-4,7-dihydro-1,3-dioxépine.

7. 2,4,4,7,7-pentaméthyl-4,7-dihydro-1,3-dioxépine.

8. 2-pentyl-4,4,7,7-tétraméthyl-4,7-dihydro-1,3-dioxépine.

9. 2-éthyl-4,4,7,7-tétraméthyl-4,7-dihydro-1,3-dioxépine.

10. 2-propyl-4,7-diméthyl-4,7-dihydro-1,3-dioxépine.

11. 2-pentyl-4,7-diméthyl-4,7-dihydro-1,3-dioxépine.